# EUROPEAN PATENT SPECIFICATION

(11) **EP 3 428 179 B1**
(45) Date of publication and mention of the grant of the patent: **25.11.2026**
(21) Application number: 17763496.1
(22) Date of filing: 27.02.2017
(51) Int. Cl.: C07K 7/06, A23L 33/18, A61K 38/08, A61P 29/00, A61P 37/00, A61P 19/02

(54) **PEPTIDE FOR PREVENTING OR TREATING INFLAMMATORY DISEASES AND USE THEREOF**
PEPTID ZUR PRÄVENTION ODER BEHANDLUNG VON ENTZÜNDLICHEN ERKRANKUNGEN UND VERWENDUNG DAVON
PEPTIDE POUR LA PRÉVENTION OU LE TRAITEMENT DE MALADIES INFLAMMATOIRES ET UTILISATION ASSOCIÉE

(30) Priority: 09.03.2016 KR 20160028229; 17.10.2016 KR 20160134177
(43) Date of publication of application: 16.01.2019
(73) Proprietor: Kine Sciences Co., Ltd., Gangnam-gu Seoul (KR)
(72) Inventor: CHO, Dae Ho, Seoul 06004 (KR); KIM, Kyung Eun, Seoul 01200 (KR); PARK, Sun Young, Yongin-si Gyeonggi-do 16860 (KR); HOUH, Youn Kyung, Seoul 08096 (KR)
(74) Representative: Ström & Gulliksson AB
(86) International application number: PCT/KR2017/002117
(87) International publication number: WO 2017/155234

(56) References cited:
- WO-A1-2013/167298
- WO-A2-02/080858
- CN-A- 1 872 873
- CN-A- 103 739 670
- JP-A- 2013 513 657
- JP-A- H0 725 783
- US-A1- 2015 299 252
- DATABASE WPI Week 199514, Derwent World Patents Index; AN 1995-101811, XP002793034
- DATABASE WPI Week 201461, Derwent World Patents Index; AN 2014-L41900, XP002793035

## Description

### Technical Field

The present invention relates to a peptide consisting of amino acid represented by SEQ ID NO: 1, to a pharmaceutical composition for use in preventing or treating inflammatory diseases as well as to a health functional food composition for use in preventing or improving an inflammatory disease.

### Background Art

Inflammation is a phenomenon that occurs for a series of defenses purposes of minimizing the response and restoring the damaged part to an original state when cells or tissues are damaged by some cause, and causes nerves and blood vessels, lymphatic vessels, body fluids, and cellular reactions, resulting in pain, swelling, redness, fever, etc., to cause dysfunction. The causes of the inflammation include physical factors caused by trauma, frostbite, burn, radioactivity, chemical factors caused by chemicals such as acid, and immunological factors due to antibody response, and in addition, caused by blood vessel or hormone imbalance. Cells damaged by external stimuli secrete a variety of biological mediators such as pro-inflammatory cytokines and chemokines, interleukins, and interferons, resulting in vasodilation and antibodies, complement, plasma, and phagocyte cells are driven into the inflammation sites as the permeability is increased. This phenomenon causes erythema. A drug that acts to remove an inflammatory source to eliminate the inflammation and to reduce vital reaction and symptoms is called an anti-inflammatory agent. Until now, substances used for the purpose of anti-inflammation include ibuprofen, indomethacin, and the like as non-steroids and dexamethasone and like as steroids, but the use thereof is limited because of the safety problem thereof. In this respect, development of safe anti-inflammatory agents with maximum efficacy and minimal side effects is required.

Arthritis, a typical inflammatory disease, is one of the most painful diseases suffered by humans and causes major disabilities in daily life due to walking disorders as well as pain.

The arthritis is classified into bacterial arthritis caused by bacteria and aseptic arthritis irrelevant to the bacteria and is known to be more than 100 kinds of arthritis.

The most common arthritis includes degenerative arthritis, rheumatoid arthritis, gouty arthritis, lupus, and gout. It is searched that 47 of 1,000 people in Korea suffer from arthritis, and it is shown that a total of 1.9 million people suffer from arthritis all over the nation. Arthritis, as a leading cause of long-term disability, is pointed out as the biggest cause of disability in the elderly over the age of 65 in the United States.

Even in Korea, arthritis is shown as a major disease of long-term disability following stroke, and it is known that women are more likely to suffer from arthritis than men.

For the treatment of arthritis, rheumatoid arthritis may be treated with drug therapy such as anti-inflammatory analgesic drugs, steroids, gold therapy, local injection of steroids, and immunosuppressive therapy, surgical therapy, and appropriate physical exercise therapy within the limits of not moving the joint too much, as well as general conservative treatments such as feeding of food and nutrients and fixing and splint. On the other hand, in the case of degenerative arthritis, appropriate exercise and rehabilitation treatment, drug therapy of administering anti-inflammatory analgesic agents, adrenocortical hormones, cartilage protecting agents, intra-articular lubricants and the like, or surgical therapy may be performed.

Drugs used in the treatment of arthritis are used to reduce pain and edema and slow the progression of the disease. However, these arthritis therapeutic agents have gastrointestinal disorders as side effects and gastric ulcers and hemorrhages when taken for a long time.

The causes for increased incidence of gastrointestinal side effects include old age over 65 years, ulceration, history of bleeding, combination of steroids or anticoagulants, and smoking or drinking. To reduce these side effects, the condition of the body after taking the medicines is observed, taking the medication immediately is stopped at any signs of gastrointestinal side effects, the treatment needs to be discussed with your doctor, and the drug needs to be changed to medicines with low gastrointestinal side effects (selective anti-inflammatory drugs, COX-2 inhibitors). The arthritis therapeutic agents used in a current clinical practice are mainly low-molecular drugs as disease-modifying antirheumatic drugs (DMARDs) such as methotrexate (MTX) as a non-steroidal anti-inflammatory drug, hydroxychloroquine and auranofin, and protein drugs such as Etanercept (Enbrel as proprietary name), Infliximab (Remicade as proprietary name) and Adalimumab (Humira as proprietary name), which have been developed as TNF-α inhibitors. Although methotrexate is a drug which is originally developed as an anticancer drug and has been currently used as an arthritis therapeutic agent, but due to toxicity to normal cells, long-term use of methotrexate causes serious side effects, and as a result, more than 50% of patients are reported to have failed treatment.

JP3059283 describes anti-inflammatory peptides of formula H-X1-Cys-A-B-A-B-A-Ser-X2-Y, A= Val, Leu, lie or Nie; B = Arg, Lys, Gin, His or Ser; X1, X2 = a bond or a peptide fraction of 1-1 O amino acid residues chosen from Gly, Ala, Val, Arg, Asn, Ser, Phe, Pro, Leu, Glu, Asp, Lys, Thr, His, Tyr, Nie and lie; Y = OH or NH2 and their use in the treatment of inflammatory diseases such as rheumatoid arthritis, periodontosis, nephritis, dermatitis, ulcerative colitis, arteriosclerosis and pulmonary silicosis.

WO2013167298 describes anti-inflammatory peptides for use in the treatment of allergies, cholecystitis, psoriasis, dermatitis, asthma, rhinitis; cystic fibrosis, arthritis, ankylosing spondylitis, allograft rejection and graft versus host disease among others.

CN103739670 describes the peptide mPEG-SC-Pro-Thr-Met-Ser-Trp for use in the treatment of rheumatoid arthritis.

WO2080858 describes the tripeptide KPV to treat cutaneous inflammation such as psoriasis and atopic dermatitis.

Under such a background, development of a novel therapeutic agent having an effective therapeutic effect while minimizing side effects of conventional anti-inflammatory agents, especially arthritis therapeutic agents, has been required and has been actively studied (Korean Patent Publication No. 10-2015-0125001), but it is still not enough. Thus, an object of the present disclosure is to provide such agents.

### Summary

### Technical Problem

In order to solve the above problems, the present inventors prepared a dimeric peptide, confirmed an excellent anti-inflammatory effect according to administration of the peptide using a mouse model of rheumatoid arthritis as a representative inflammatory disease, and completed the present invention based thereon.

One aspect of the present invention relates to a peptide consisting of amino acid represented by SEQ ID NO: 1.

A second aspect of the present invention relates to a pharmaceutical composition for use in preventing or treating an inflammatory disease, containing a peptide consisting of amino acid represented by SEQ ID NO: 1 or a polynucleotide encoding the peptide as an active ingredient.

A third aspect of the present invention relates to a health functional food composition for use in preventing or improving an inflammatory disease, containing a peptide consisting of amino acid represented by SEQ ID NO: 1 or a polynucleotide encoding the peptide as an active ingredient.

### Technical Solution

In order to achieve the object, in one aspect, the present invention provides a peptide consisting of amino acid represented by SEQ ID NO: 1.

In a preferred embodiment, an N- or C-terminal of the peptide is bound to a protective group selected from the group consisting of an acetyl group, a fluorenylmethoxy carbonyl group, a formyl group, a palmitoyl group, a myristyl group, a stearyl group or a polyethylene glycol (PEG) group.

In another aspect, the present invention provides a pharmaceutical composition for use in preventing or treating an inflammatory disease, containing a peptide consisting of amino acid represented by SEQ ID NO: 1 or a polynucleotide encoding the peptide as an active ingredient.

In a preferred embodiment, an N- or C-terminal of the peptide is bound to a protective group selected from the group consisting of an acetyl group, a fluorenylmethoxy carbonyl group, a formyl group, a palmitoyl group, a myristyl group, a stearyl group or a polyethylene glycol (PEG) group.

In a preferred embodiment, the inflammatory disease is selected from the group consisting of psoriasis, dermatitis, allergies, arthritis, rhinitis, otitis media, sore throat, tonsillitis, cystitis, nephritis, pelvic inflammatory disease, Crohn's disease, ulcerative colitis, ankylosing spondylitis, systemic lupus erythematodes (SLE), asthma, edema, delayed allergy (IV type allergy), graft rejection, graft versus host disease, autoimmune encephalopathy, multiple sclerosis, inflammatory bowel disease, cystic fibrosis, diabetic retinopathy, ischemia-reperfusion injury, vascular restenosis, glomerulonephritis, and gastrointestinal allergy.

In a further preferred embodiment, the arthritis is selected from the group consisting of osteoarthritis, degenerative arthritis, rheumatoid arthritis, osteochondritis dissecans, joint ligament injury, medial meniscus injury, malalignment of joints, avascular necrosis, and juvenile idiopathic arthritis.

In still another preferred embodiment, the pharmaceutical composition further comprises a pharmaceutically acceptable carrier.

In yet another preferred embodiment, the pharmaceutical composition is simultaneously, separately or sequentially administered with other anti-inflammatory agents in addition to the peptide consisting of amino acid represented by SEQ ID NO: 1 or the polynucleotide encoding the peptide.

In still yet another preferred embodiment, the pharmaceutical composition is formulated for oral administration, intramuscular administration, intravenous administration, intraperitoneal administration, subcutaneous administration, intradermal administration, or topical administration.

In a second aspect, the present invention provides a health functional food composition for use preventing or improving an inflammatory disease, containing a peptide consisting of amino acid represented by SEQ ID NO: 1 or a polynucleotide encoding the peptide as an active ingredient.

In a preferred embodiment, the inflammatory disease is selected from the group consisting of psoriasis, dermatitis, allergies, arthritis, rhinitis, otitis media, sore throat, tonsillitis, cystitis, nephritis, pelvic inflammatory disease, Crohn's disease, ulcerative colitis, ankylosing spondylitis, systemic lupus erythematodes (SLE), asthma, edema, delayed allergy (IV type allergy), graft rejection, graft versus host disease, autoimmune encephalopathy, multiple sclerosis, inflammatory bowel disease, cystic fibrosis, diabetic retinopathy, ischemia-reperfusion injury, vascular restenosis, glomerulonephritis, and gastrointestinal allergy.

### Advantageous Effects

Through the novel dimeric peptide according to the present invention, it is possible to not only provide an excellent therapeutic effect through anti-inflammatory action but also have a very small-sized peptide, thereby minimizing side effects due to the administration of external substances. The peptide can be expected to be used as an active substance that can replace existing therapeutic agents for inflammatory diseases.

### Description of Drawings

FIG. 1 is a schematic diagram schematically illustrating a preparation process of a collagen-induced arthritis mouse model and an administration timing of a peptide (a RDG dimer) of the present invention.
FIG. 2 illustrates a result of periodically measuring an arthritic progression index according to the peptide treatment after induction of a second immunity response in the collagen-induced arthritis mouse model (Normal: normal mouse, Vehicle control: control mouse, and RDG dimer: mouse treated with RDG dimer).
FIG. 3 illustrates a result of visually confirming a mitigating effect of edema and redness around the ankle joint according to the peptide treatment of the present invention.
FIG. 4 illustrates a result of periodically measuring an arthritic progression index according to the peptide treatment at various concentrations (0.1 µmole, 1 µmole, and 10 µmole) after induction of secondary immune response in the collagen-induced arthritis mouse model (Normal: normal mouse, Vehicle: control mouse, RDG dimer 0.1 µmole: mouse treated with 0.1 µmole of RDG dimer, RDG dimer 1 µmole: mouse treated with 1 µmole of RDG dimer, RDG dimer 10 µmole: mouse treated with 10 µmole of RDG dimer, and MTX: mouse treated with methotrexate).

### Modes of the Invention

Hereinafter, the present invention will be described in detail.

The present invention provides a peptide consisting of amino acid represented by SEQ ID NO: 1.

The peptide of the present invention may additionally include an amino acid sequence prepared for the specific purpose of increasing a targeting sequence, a tag, a labeled residue, half-life, or peptide stability.

In addition, the peptide of the present invention may be obtained by various methods well known in the art. For example, the peptide may be prepared by polynucleotide recombination and protein expression systems or synthesis in vitro through chemical synthesis such as peptide synthesis, and cell-free protein synthesis.

In addition, to obtain better chemical stability, enhanced pharmacological properties (half-life, absorbency, potency, efficacy, etc.), altered specificity (e.g., broad biological activity spectrum), and reduced antigenicity, a protective group may be bound to an N- or C-terminal of the peptide. Preferably, the protective group may be an acetyl group, a fluorenylmethoxycarbonyl group, a formyl group, a palmitoyl group, a myristyl group, a stearyl group or a polyethylene glycol (PEG) group, but may include any ingredient capable of enhancing modification of the peptide, particularly, stability of the peptide. The term "stability" used in the present invention refers to storage stability (for example, room-temperature storage stability) as well as in-vivo stability that protects the peptide of the present invention from attack of a protein cleavage enzyme in vivo.

In the present invention, "polynucleotide" is a polymer to which a nucleotide binds, and serves to transmit genetic information. For the purpose of the present invention, the polynucleotide encodes the peptide of SEQ ID NO: 1.

The term "homology" is to indicate a similar degree to a wide-type amino acid sequence or a polynucleotide sequence, and the comparison of the homology may be performed using a comparison program which is well-known in the art and homology between at least two sequences may be calculated by a percentage (%).

In another aspect of the present invention, the present invention provides a pharmaceutical composition for use in preventing or treating an inflammatory disease, containing a peptide consisting of amino acid represented by SEQ ID NO: 1 or a polynucleotide encoding the peptide as an active ingredient.

The term "prevention" used in the present invention means all actions for inhibiting the inflammatory disease or delaying the onset by administering the pharmaceutical composition according to the present invention.

The term "treatment" used in the present invention means all actions for improving or beneficially changing symptoms for the inflammatory disease by administering the pharmaceutical composition according to the present invention.

In the present invention, the "subject" refers to a subject requiring treatment of an inflammatory disease, and more specifically, refers to human or non-human primates, and mammals such as mouse, dogs, cats, horses, and cows.

The "inflammatory disease" which is a disease to be prevented or treated by the composition of the present invention refers to a generic term of a disease having inflammation as a lesion. The inflammatory disease may be psoriasis, dermatitis, allergies, arthritis, rhinitis, otitis media, sore throat, tonsillitis, cystitis, nephritis, pelvic inflammatory disease, Crohn's disease, ulcerative colitis, ankylosing spondylitis, systemic lupus erythematodes (SLE), asthma, edema, delayed allergy (IV type allergy), graft rejection, graft versus host disease, autoimmune encephalopathy, multiple sclerosis, inflammatory bowel disease, cystic fibrosis, diabetic retinopathy, ischemia-reperfusion injury, vascular restenosis, glomerulonephritis, or gastrointestinal allergy. Herein, arthritis refers to a disease caused by a inflammatory change in the joints due to various causes such as bacteria, trauma, autoimmune diseases, and the like, and may be preferably osteoarthritis, degenerative arthritis, rheumatoid arthritis, osteochondritis dissecans, joint ligament injury, medial meniscus injury, malalignment of joints, avascular necrosis, and juvenile idiopathic arthritis and most preferably rheumatoid arthritis.

The peptide of the present invention, a dimeric peptide, was prepared using RDG monomers (see Example 1), and a therapeutic effect (an anti-inflammatory activity) was confirmed by a decrease in arthritis progression index using a mouse model of rheumatoid arthritis, a typical inflammatory disease, and through a comparison with methotrexate as a conventional arthritis therapeutic agent, and a treatment concentration (0.1 to 1 µmole) of the peptide which exhibits the optimal therapeutic effect was found (see Example 2).

The term "anti-inflammatory activity" used in the present invention refers to inhibition of the inflammatory response, and the inflammation, which is one of defensive responses of biological tissues to any stimuli, refers to a complicated lesion which is developed together with tissue degeneration, circulatory disturbance and exudation, and tissue proliferation.

The peptide of the present invention, as one of these anti-inflammatory activities, may inhibit generation of inflammatory cytokine.

The peptide or the polynucleotide encoding the peptide of the present invention may be delivered to a pharmaceutically acceptable carrier such as a colloidal suspension, powder, saline, lipid, liposome, microspheres, or nanospheric particles. The peptide or the polynucleotide may form a complex with a carrier or may be associated with the carrier and may be delivered in vivo using a delivery system which is known in the art, such as lipids, liposomes, microparticles, gold, nanoparticles, polymers, condensation reagents, polysaccharides, polyamino acids, dendrimers, saponins, adsorption enhancers or fatty acids.

The pharmaceutically acceptable carrier includes lactose, dextrose, sucrose, sorbitol, mannitol, starch, acacia, rubber, calcium phosphate, alginate, gelatin, calcium silicate, microcrystalline cellulose, polyvinylpyrrolidone, cellulose, water, syrup, methylcellulose, hydroxybenzoate, propylhydroxybenzoate, talc, magnesium stearate, mineral oil, and the like, which are generally used in formulation. Further, the pharmaceutical composition may further include lubricants, wetting agents, sweeteners, flavors, emulsifiers, suspensions, preservatives, and the like in addition to the ingredients.

The pharmaceutical composition of the present invention may be administered orally or parenterally (for example, intramuscularly, intravenously, intraperitoneally, subcutaneously, intradermally, or topically) depending on the intended method, and the dose varies according to the condition and weight of a patient, the degree of disease, a type of drug, and the route and time of administration, but may be suitably selected by those skilled in the art.

The pharmaceutical composition may be administered with a pharmaceutically effective dose, for example, 0.1 to 10 µmol. The "pharmaceutically effective dose" refers to a amount which is sufficient to treat the diseases at a reasonable benefit/risk ratio applicable to medical treatment, and an effective dose level may be determined according to elements including a kind of disease of the patient, the severity, activity of a drug, sensitivity to a drug, a time of administration, a route of administration, and an emission rate, duration of treatment, and simultaneously used drugs and other elements well-known in the medical field.

The pharmaceutical composition according to the present invention may be administered as an individual therapeutic agent or in combination with other anti-inflammatory agents, simultaneously, separately or sequentially administered with existing anti-inflammatory agents, and administered singly or multiply. It is important to administer an amount capable of obtaining a maximum effect with a minimal amount without side effects by considering all of the elements and the amount may be easily determined by those skilled in the art.

In particular, the effective dose of the pharmaceutical composition may vary according to age, gender, condition, and weight of the patient, absorption of active ingredients in the body, inactive rate, excretion rate, disease type, and combined drugs, and may be increased or decreased according to the route of administration, the severity of obesity, gender, weight, age, and the like.

In another aspect of the present invention, the present invention provides a health functional food composition for use in preventing or improving an inflammatory disease, containing a peptide consisting of amino acid represented by SEQ ID NO: 1 or a polynucleotide encoding the peptide as an active ingredient.

The term "improvement" used in the present invention means all actions that at least reduce parameters associated with a treated condition, for example, the degree of symptoms. At this time, the health functional food composition may be used simultaneously or separately with the drug for treatment before or after the onset of the corresponding disease for preventing or improving the inflammatory disease.

In the health functional food composition of the present invention, the active ingredient may be added to the food as it is or may be used together with other food or food ingredients, and may be appropriately used according to general methods. A mixing amount of the active ingredients may be appropriately determined according to a purpose of use (for prevention or improvement) thereof. Generally, in preparation of foods or beverages, the composition of the present invention may be added with an amount of preferably 15 wt% or less and more preferably 10 wt% or less with respect to a raw material. However, in the case of long-term ingestion for the purpose of health and hygiene or health regulation, the amount may be below the above range.

The health functional food composition of the present invention may contain other ingredients as a required ingredient without specific limitation other than the active ingredient. For example, like a general beverage, various flavoring agents or natural carbohydrates may be added as an additional ingredient. Examples of the aforementioned natural carbohydrates include general sugars, such as monosaccharides, for example, glucose, fructose, and the like; disaccharides, for example, maltose, sucrose, and the like; and polysaccharides, for example, dextrin, cyclodextrin, and the like; and sugar alcohols, such as xylitol, sorbitol, and erythritol. As the flavoring agents other than the above examples, natural flavoring agents (thaumatin and stevia extract (e.g., rebaudioside A, glycyrrhizin, etc.) and synthetic flavoring agents (saccharin, aspartame, etc.) may be advantageously used. The ratio of the natural carbohydrate may be appropriately determined by selection of those skilled in the art.

In addition, the health food composition according to the present invention may contain various nutrients, vitamins, minerals (electrolytes), flavoring agents such as synthetic flavoring agents and natural flavoring agents, coloring agents and thickening agents (cheese, chocolate, etc.), pectic acid and salts thereof, alginic acid and salts thereof, organic acid, a protective colloidal thickener, a pH adjusting agent, a stabilizer, a preservative, glycerin, alcohol, a carbonic acid agent used in a carbonated drink, and the like. These ingredients may be used independently or in combination, and the ratio of such additives may also be appropriately selected by those skilled in the art.

Hereinafter, preferred Examples for helping in understanding of the present invention are proposed. The following Examples are provided for more easily understanding the present invention.

### Examples

The references to methods of treatment by therapy in the examples are to be interpreted as references to peptides, pharmaceutical compositions and health functional food compositions of the present invention for use in those methods.

### Example 1. Preparation of peptide

In Example 1, a RDG dimeric polymer (RDGRDG (SEQ ID NO: 1)) was prepared using RDG monomers. Thereafter, the synthesized peptide was purified using a high-performance liquid chromatography (SHIMADZU Prominence HPLC) and a column used a Shiseido capcell pak C18 column (4.6 x 50 mm). Further, the mass of the synthesized peptide was confirmed using a mass spectrometer (HP 1100 series LC/MSD).

### Example 2. Analysis of arthritis therapeutic effect using collagen-induced arthritis mouse model

In Example 2, using a collagen-induced arthritis mouse model in the following document, a therapeutic effect of a RGD dimer for a representative inflammatory disease, rheumatoid arthritis was confirmed (Nat Protoc. 2007;2(5):1269-75.).

### 2-1. Preparation of collagen-induced arthritis mouse model and administration of peptide

A collagen-induced arthritis (CIA) mouse model was prepared as follows, as an autoimmune disease-based arthritis model with characteristics similar to human rheumatoid arthritis.

First, a bovine type II collagen (Chondrex Corporation, USA) and a Freund's complete adjuvant (Chondrex Corporation, USA) were mixed at a ratio of 1:1 and emulsified, and then 50 µl of the emulsified collagen solution was injected intradermally into the tail of a 6-week-old DBA/1J mouse to induce primary immunization. At two weeks after the primary immunization, the bovine type II collagen and the Freund's complete adjuvant were mixed at a ratio of 1:1 and emulsified, and then 50 µl of the emulsified collagen solution was injected intradermally into the tail of the mouse again to induce secondary immunization (boosting).

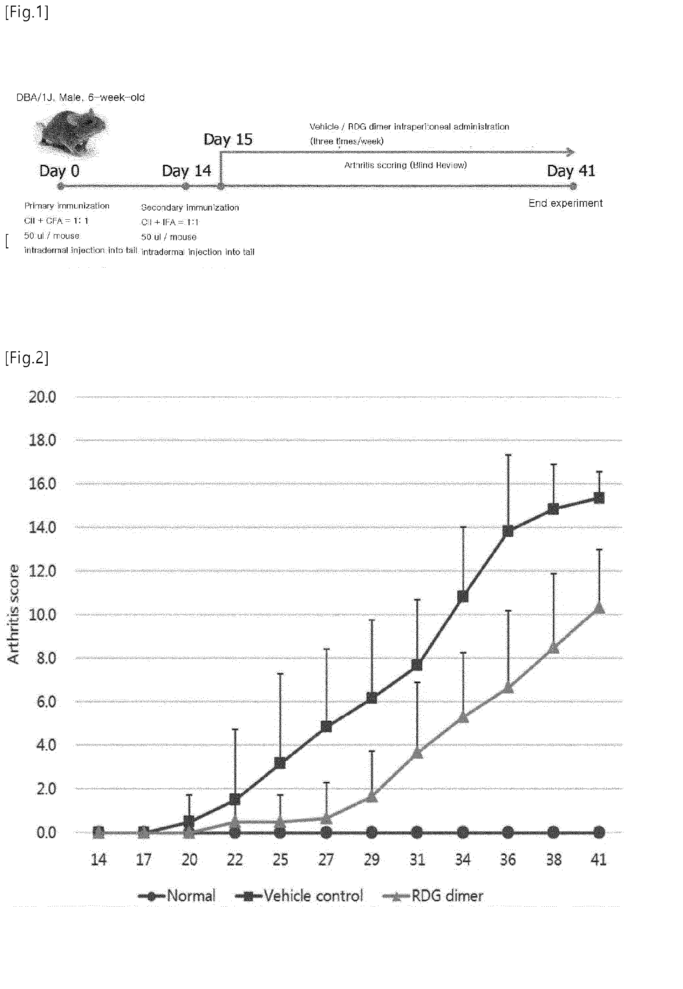

After the secondary immunization, from the next day, the peptide of the present invention (RDG dimer (1 µmole)) was administered intraperitoneally three times a week and a PBS-treated group was used as a control (Vehicle control). The preparation process of the collagen-induced arthritis mouse model and the administration time of the peptide were schematically illustrated in FIG. 1.

### 2-2. Evaluation of deepening degree of arthritis

In order to investigate the development of arthritis progression according to the peptide treatment of the present invention, the deepening degree of arthritis with time was measured by evaluating an arthritis progression index. Two observers who were unaware of the specific experimental conditions evaluated progression of arthritis three times a week. In this case, the arthritis progression index was evaluated by 0 to 4 scores per leg according to an arthritis progression evaluation standard by Rossoliniec and the like in Table 2 below to be represented by a total of 0 to 16 scores (a sum of four legs), and thereafter, two observers calculated an average value of the evaluated results to digitize the severity of arthritis.

**Table 1**

| **Score** | **symptoms** |
|---|---|
| 0 | There is no edema or swelling. |
| 1 | Slight edema and redness limited to foot or ankle joint are observed. |
| 2 | Slight edema and redness from the ankle joint to the tarsal bone are observed. |
| 3 | Moderate edema and redness from the ankle joint to the tarsal bone are observed. |
| 4 | Edema and redness from the ankle to the entire leg are present and joint stiffness is observed. |

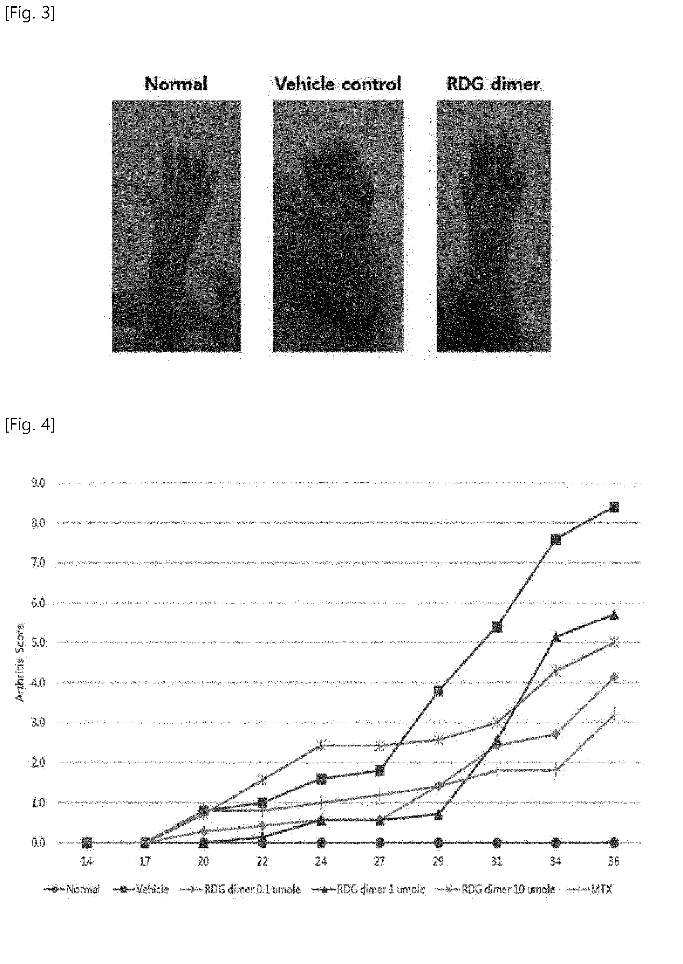

As a result, as illustrated in Table 2 below and FIG. 2, it could be seen that in a control mouse (Vehicle control, PBS), on 41 day, the arthritis progressed so that the arthritis progression index reached 15.3, whereas in the RDG dimer-treated group, the arthritis progression index which has significantly increased in the control mouse was significantly decreased to 10.3. Actually, as the result of visually checking the degree of swelling and redness around the ankle joint, as illustrated in FIG. 3, it could be seen that in the RDG dimer-treated group, the swelling and redness symptoms were remarkably mitigated as compared with the control.

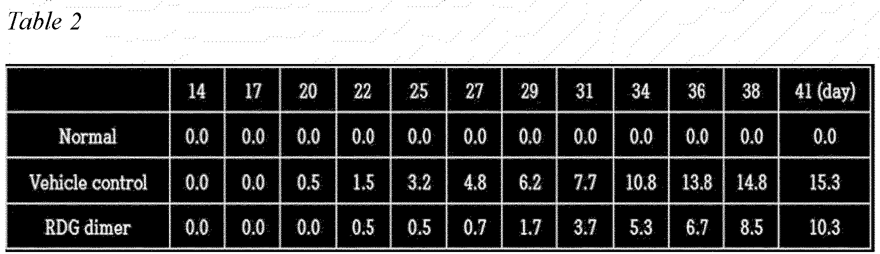

### 2-3. Confirmation of efficacy for each treatment concentration of peptide

In order to confirm the efficacy for each treatment concentration of the peptide according to the present invention, by using the same method as Example 2-2, an arthritis therapeutic effect according to the peptide treatment at various concentrations (0.1 µmole, 1 µmole, and 10 µmole) was evaluated by the arthritis progression index.

Methotrexate (MTX), one of drugs which are used as arthritis therapeutic agents in a current clinical practice, had excellent anti-inflammatory and arthritis therapeutic effects as an immunodepressant, but may have side effects such as toxicity to normal cells, oral ulcer, alopecia, interstitial pneumonia, bone marrow suppression, and the like. As a result, the methotrexate is known as a drug that needs to pay close attention after medication with sufficient medication counseling. As a result, the arthritis therapeutic effect according to the peptide treatment at various concentrations was compared with that of the case of treating methotrexate (1 mg/kg).

As a result, as illustrated in Table 3 below and FIG. 4, the arthritis progression index which had significantly increased in the control mouse was significantly decreased at all the concentrations in the case of treating the RDG dimer, and among them, in the case of treating the RDG dimer at a concentration of 0.1 µmole, the effect was remarkable (on 36 day: 4.1). Further, the effect was approximate to the case of treating methotrexate, and as a result, the excellent arthritis therapeutic effect was confirmed again.

**Table 3**

| | **14** | **17** | **20** | **22** | **24** | **27** | **29** | **31** | **34** | **36 (day)** |
|---|---|---|---|---|---|---|---|---|---|---|
| **Normal** | **0.0** | **0.0** | **0.0** | **0.0** | **0.0** | **0.0** | **0.0** | **0.0** | **0.0** | **0.0** |
| **Vehicle control** | **0.0** | **0.0** | **0.8** | **1.0** | **1.6** | **1.8** | **3.8** | **5.4** | **7.6** | **8.4** |
| **RDG dimer 0.1 ᵤmole** | **0.0** | **0.0** | **0.3** | **0.4** | **0.6** | **0.0** | **1.4** | **2.4** | **2.7** | **4.1** |
| **RDG dimer 1 ᵤmolo** | **0.0** | **0.0** | **0.0** | **0.1** | **0.6** | **0.6** | **0.7** | **2.6** | **5.1** | **5.7** |
| **RDG dimer 10 ᵤmole** | **0.0** | **0.0** | **0.7** | **1.6** | **2.4** | **2.4** | **2.6** | **3.0** | **4.3** | **5.0** |
| **MTX** | **0.0** | **0.0** | **0.8** | **0.8** | **1.0** | **1.2** | **1.4** | **1.8** | **1.8** | **3.2** |

### Example 3. Evaluation of toxicity

In Example 3, in order to confirm the toxicity of the **peptide** of the present invention, the RGD dimer was intraperitoneally administered to mouse at various concentrations (0.1 µmole, 1 µmole, and 10 µmole), and then the mortality and a change in body weight of each mouse were measured. On the other hand, as a control, a non-administration group (Normal) and a PBS-treated group (PBS) were used.

As a result, even at the concentration of 1 µmole or 10 µmole in addition to the concentration of 0.1 µmole which had the excellent arthritis therapeutic effect in Example 2-3, the mortality of the mouse according to the peptide treatment was not observed. As illustrated in Table 4 below, at all the concentrations, the mouse did not have a large difference in body weight from that of the control, and as a result, it can be seen that the peptide of the present invention is a safe ingredient which does not cause the toxicity in vivo.

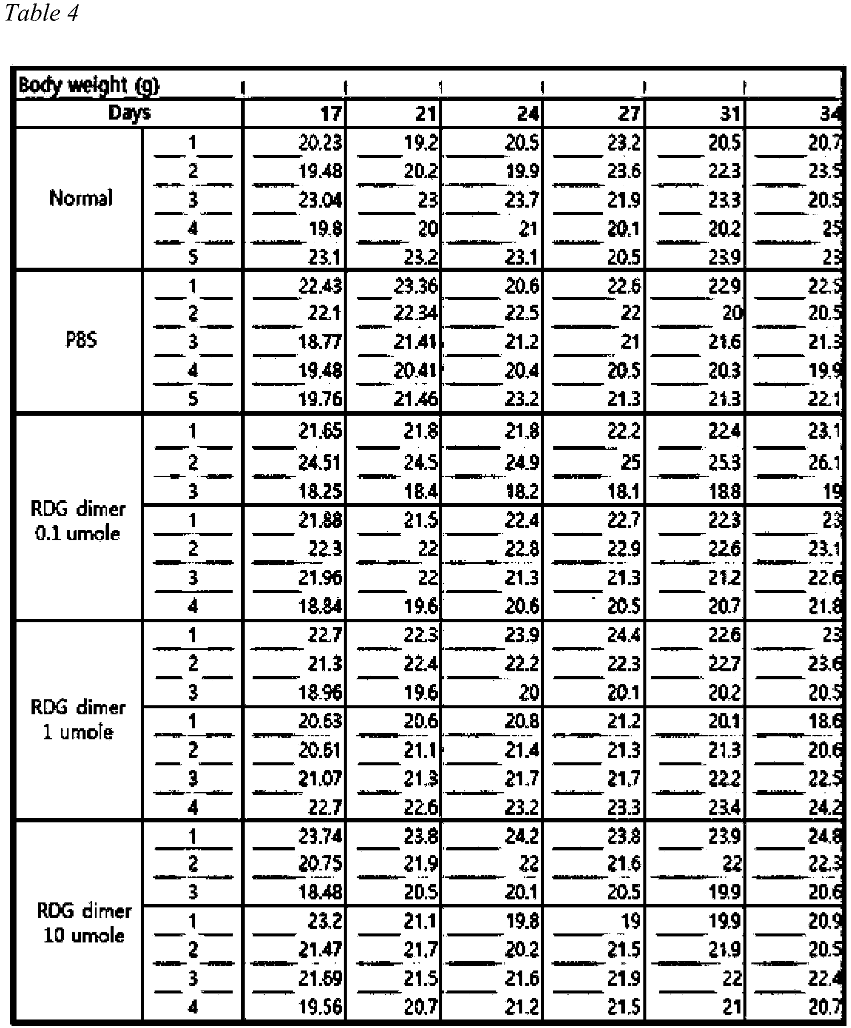

When describing the results, it can be seen that the peptide (the RDG dimer) of the present invention may effectively mitigate the inflammatory response of the typical inflammatory disease, rheumatoid arthritis, and may be used as an active substance for the treatment of the inflammatory diseases.

## Claims

1. A peptide consisting of amino acid represented by SEQ ID NO: 1.

2. The peptide of claim 1, wherein an N- or C-terminal of the peptide is bound to a protective group selected from the group consisting of an acetyl group, a fluorenylmethoxy carbonyl group, a formyl group, a palmitoyl group, a myristyl group, a stearyl group or a polyethylene glycol (PEG) group.

3. A pharmaceutical composition for use in preventing or treating an inflammatory disease, containing a peptide consisting of amino acid represented by SEQ ID NO: 1 or a polynucleotide encoding the peptide as an active ingredient.

4. The pharmaceutical composition for use according to claim 3, wherein an N- or C-terminal of the peptide is bound to a protective group selected from the group consisting of an acetyl group, a fluorenylmethoxy carbonyl group, a formyl group, a palmitoyl group, a myristyl group, a stearyl group or a polyethylene glycol (PEG) group.

5. The pharmaceutical composition for use according to claim 3, wherein the inflammatory disease is selected from the group consisting of psoriasis, dermatitis, allergies, arthritis, rhinitis, otitis media, sore throat, tonsillitis, cystitis, nephritis, pelvic inflammatory disease, Crohn's disease, ulcerative colitis, ankylosing spondylitis, systemic lupus erythematodes (SLE), asthma, edema, delayed allergy (IV type allergy), graft rejection, graft versus host disease, autoimmune encephalopathy, multiple sclerosis, inflammatory bowel disease, cystic fibrosis, diabetic retinopathy, ischemia-reperfusion injury, vascular restenosis, glomerulonephritis, and gastrointestinal allergy.

6. The pharmaceutical composition for use according to claim 5, wherein the arthritis is selected from the group consisting of osteoarthritis, degenerative arthritis, rheumatoid arthritis, osteochondritis dissecans, joint ligament injury, medial meniscus injury, malalignment of joints, avascular necrosis, and juvenile idiopathic arthritis.

7. The pharmaceutical composition for use according to claim 3, further comprising: a pharmaceutically acceptable carrier.

8. The pharmaceutical composition for use according to claim 3, wherein the composition is simultaneously, separately or sequentially administered with other anti-inflammatory agents in addition to the peptide consisting of amino acid represented by SEQ ID NO: 1 or the polynucleotide encoding the peptide.

9. The pharmaceutical composition for use according to claim 3, wherein the composition is formulated for oral administration, intramuscular administration, intravenous administration, intraperitoneal administration, subcutaneous administration, intradermal administration, or topical administration.

10. A health functional food composition for use in preventing or improving an inflammatory disease, containing a peptide consisting of amino acid represented by SEQ ID NO: 1 or a polynucleotide encoding the peptide as an active ingredient.

11. The health functional food composition for use according to claim 10, wherein the inflammatory disease is selected from the group consisting of psoriasis, dermatitis, allergies, arthritis, rhinitis, otitis media, sore throat, tonsillitis, cystitis, nephritis, pelvic inflammatory disease, Crohn's disease, ulcerative colitis, ankylosing spondylitis, systemic lupus erythematodes (SLE), asthma, edema, delayed allergy (IV type allergy), graft rejection, graft versus host disease, autoimmune encephalopathy, multiple sclerosis, inflammatory bowel disease, cystic fibrosis, diabetic retinopathy, ischemia-reperfusion injury, vascular restenosis, glomerulonephritis, and gastrointestinal allergy.

## Patentansprüche

1. Peptid, bestehend aus der durch SEQ ID NO: 1 dargestellten Aminosäuresequenz.

2. Peptid nach Anspruch 1, wobei ein N- oder C-Terminus des Peptids mit einer Schutzgruppe verbunden ist, die ausgewählt ist aus der Gruppe bestehend aus einer Acetylgruppe, einer Fluorenylmethoxycarbonylgruppe, einer Formylgruppe, einer Palmitoylgruppe, einer Myristylgruppe, einer Stearylgruppe oder einer Polyethylenglykol-(PEG)-Gruppe.

3. Pharmazeutische Zusammensetzung zur Verwendung bei der Prävention oder Behandlung einer entzündlichen Erkrankung, enthaltend als Wirkstoff ein Peptid, bestehend aus der durch SEQ ID NO: 1 dargestellten Aminosäuresequenz, oder ein das Peptid codierendes Polynukleotid.

4. Pharmazeutische Zusammensetzung zur Verwendung nach Anspruch 3, wobei ein N- oder C-Terminus des Peptids mit einer Schutzgruppe verbunden ist, die ausgewählt ist aus der Gruppe bestehend aus einer Acetylgruppe, einer Fluorenylmethoxycarbonylgruppe, einer Formylgruppe, einer Palmitoylgruppe, einer Myristylgruppe, einer Stearylgruppe oder einer Polyethylenglykol-(PEG)-Gruppe.

5. Pharmazeutische Zusammensetzung zur Verwendung nach Anspruch 3, wobei die entzündliche Erkrankung ausgewählt ist aus der Gruppe bestehend aus Psoriasis, Dermatitis, Allergien, Arthritis, Rhinitis, Otitis media, Halsschmerzen, Tonsillitis, Zystitis, Nephritis, entzündlichen Erkrankungen des Beckens, Morbus Crohn, Colitis ulcerosa, ankylosierender Spondylitis, systemischem Lupus erythematodes (SLE), Asthma, Ödemen, verzögerter Allergie (Typ-IV-Allergie), Transplantatabstoßung, Graft-versus-Host-Erkrankung, autoimmuner Enzephalopathie, multipler Sklerose, entzündlichen Darmerkrankungen, Mukoviszidose, diabetischer Retinopathie, Ischämie-Reperfusionsschäden, vaskulärer Restenose, Glomerulonephritis und gastrointestinaler Allergie.

6. Pharmazeutische Zusammensetzung zur Verwendung nach Anspruch 5, wobei die Arthritis ausgewählt ist aus der Gruppe bestehend aus Osteoarthritis, degenerativer Arthritis, rheumatoider Arthritis, Osteochondrosis dissecans, Verletzungen von Gelenkbändern, Verletzungen des Innenmeniskus, Fehlstellungen von Gelenken, avaskulärer Nekrose und juveniler idiopathischer Arthritis.

7. Pharmazeutische Zusammensetzung zur Verwendung nach Anspruch 3, ferner aufweisend einen pharmazeutisch annehmbaren Träger.

8. Pharmazeutische Zusammensetzung zur Verwendung nach Anspruch 3, wobei die Zusammensetzung zusätzlich zu dem Peptid, bestehend aus der durch SEQ ID NO: 1 dargestellten Aminosäuresequenz, oder dem das Peptid codierenden Polynukleotid gleichzeitig, getrennt oder nacheinander mit anderen entzündungshemmenden Mitteln verabreicht wird.

9. Pharmazeutische Zusammensetzung zur Verwendung nach Anspruch 3, wobei die Zusammensetzung für die orale Verabreichung, intramuskuläre Verabreichung, intravenöse Verabreichung, intraperitoneale Verabreichung, subkutane Verabreichung, intradermale Verabreichung oder topische Verabreichung formuliert ist.

10. Zusammensetzung eines funktionellen Lebensmittels zur Verwendung bei der Prävention oder Verbesserung einer entzündlichen Erkrankung, enthaltend als Wirkstoff ein Peptid, bestehend aus der durch SEQ ID NO: 1 dargestellten Aminosäuresequenz, oder ein das Peptid codierendes Polynukleotid.

11. Zusammensetzung eines funktionellen Lebensmittels zur Verwendung nach Anspruch 10, wobei die entzündliche Erkrankung ausgewählt ist aus der Gruppe bestehend aus Psoriasis, Dermatitis, Allergien, Arthritis, Rhinitis, Otitis media, Halsschmerzen, Tonsillitis, Zystitis, Nephritis, entzündlichen Erkrankungen des Beckens, Morbus Crohn, Colitis ulcerosa, ankylosierender Spondylitis, systemischem Lupus erythematodes (SLE), Asthma, Ödemen, verzögerter Allergie (Typ-IV-Allergie), Transplantatabstoßung, Graft-versus-Host-Erkrankung, autoimmuner Enzephalopathie, multipler Sklerose, entzündlichen Darmerkrankungen, Mukoviszidose, diabetischer Retinopathie, Ischämie-Reperfusionsschäden, vaskulärer Restenose, Glomerulonephritis und gastrointestinaler Allergie.

## Revendications

1. Peptide constitué d'acides aminés représentés par SEQ ID NO: 1.

2. Peptide selon la revendication 1, dans lequel une extrémité N-terminale ou C-terminale du peptide est liée à un groupe protecteur choisi dans le groupe consistant en un groupe acétyle, un groupe fluorénylméthoxycarbonyle, un groupe formyle, un groupe palmitoyle, un groupe myristyle, un groupe stéaryle ou un groupe polyéthylène glycol (PEG).

3. Composition pharmaceutique pour son utilisation dans la prévention ou le traitement d'une maladie inflammatoire, contenant un peptide constitué d'acides aminés représentés par SEQ ID NO: 1 ou un polynucléotide codant pour le peptide en tant que principe actif.

4. Composition pharmaceutique pour son utilisation selon la revendication 3, dans laquelle une extrémité N-terminale ou C-terminale du peptide est liée à un groupe protecteur choisi dans le groupe consistant en un groupe acétyle, un groupe fluorénylméthoxycarbonyle, un groupe formyle, un groupe palmitoyle, un groupe myristyle, un groupe stéaryle ou un groupe polyéthylène glycol (PEG).

5. Composition pharmaceutique pour son utilisation selon la revendication 3, dans laquelle la maladie inflammatoire est choisie dans le groupe consistant en psoriasis, dermatite, allergies, arthrite, rhinite, otite moyenne, mal de gorge, amygdalite, cystite, néphrite, maladie inflammatoire pelvienne, maladie de Crohn, colite ulcéreuse, spondylarthrite ankylosante, lupus érythémateux systémique (LES), asthme, œdème, allergie retardée (allergie de type IV), rejet de greffe, maladie du greffon contre l'hôte, encéphalopathie auto-immune, sclérose en plaques, maladie inflammatoire de l'intestin, mucoviscidose, rétinopathie diabétique, lésion d'ischémie-reperfusion, resténose vasculaire, glomérulonéphrite et allergie gastro-intestinale.

6. Composition pharmaceutique pour son utilisation selon la revendication 5, dans laquelle l'arthrite est choisie dans le groupe consistant en arthrose, arthrite dégénérative, polyarthrite rhumatoïde, ostéochondrite disséquante, lésion des ligaments articulaires, lésion du ménisque médial, désalignement des articulations, nécrose avasculaire et arthrite juvénile idiopathique.

7. Composition pharmaceutique pour son utilisation selon la revendication 3, comprenant en outre : un excipient pharmaceutiquement acceptable.

8. Composition pharmaceutique pour son utilisation selon la revendication 3, dans laquelle la composition est administrée simultanément, séparément ou séquentiellement avec d'autres agents anti-inflammatoires en plus du peptide constitué d'acides aminés représentés par SEQ ID NO: 1 ou du polynucléotide codant pour le peptide.

9. Composition pharmaceutique pour son utilisation selon la revendication 3, dans laquelle la composition est formulée pour une administration orale, une administration intramusculaire, une administration intraveineuse, une administration intrapéritonéale, une administration sous-cutanée, une administration intradermique ou une administration topique.

10. Composition alimentaire fonctionnelle pour la santé pour son utilisation dans la prévention ou l'amélioration d'une maladie inflammatoire, contenant un peptide constitué d'acides aminés représentés par SEQ ID NO: 1 ou un polynucléotide codant pour le peptide en tant que principe actif.

11. Composition alimentaire fonctionnelle pour la santé pour son utilisation selon la revendication 10, dans laquelle la maladie inflammatoire est choisie dans le groupe consistant en psoriasis, dermatite, allergies, arthrite, rhinite, otite moyenne, mal de gorge, amygdalite, cystite, néphrite, maladie inflammatoire pelvienne, maladie de Crohn, colite ulcéreuse, spondylarthrite ankylosante, lupus érythémateux systémique (LES), asthme, œdème, allergie retardée (allergie de type IV), rejet de greffe, maladie du greffon contre l'hôte, encéphalopathie auto-immune, sclérose en plaques, maladie inflammatoire de l'intestin, mucoviscidose, rétinopathie diabétique, lésion d'ischémie-reperfusion, resténose vasculaire, glomérulonéphrite et allergie gastro-intestinale.
